# EUROPEAN PATENT APPLICATION

(11) **EP 3 293 193 A2**
(43) Date of publication of application: **14.03.2018**
(21) Application number: 17190072.3
(22) Date of filing: 13.08.2013
(51) Int. Cl.: C07F 9/90, C07F 9/94, C07C 395/00, C23C 16/18

(54) **PRECURSORS FOR GST FILMS IN ALD/CVD PROCESSES**

(30) Priority: 13.08.2012 US 201213572973
(62) Divisional of application: 13180256.3
(71) Applicant: Versum Materials US, LLC, Tempe, AZ 85284 (US)
(72) Inventor: XIAO, Manchao, San Diego, CA California 92130 (US); BUCHANAN, Iain, Stirling, FK7 9LB (GB); LEI, Xinjian, Vista, CA California 92081 (US)
(74) Representative: Beck Greener

(57) **Abstract**

A process of making an antimony-containing or bismuth containing film such as a germanium-antimony-tellurium alloy (GST) or germanium-bismuth-tellurium (GBT) film uses a process selected from atomic layer deposition and chemical vapor deposition. A silylantimony precursor or silylbismuth precursor is used as a source of antimony or bismuth.

## Description

### BACKGROUND OF THE INVENTION

As an emerging technology, phase change materials attract more and more interest for their applications in manufacturing a new type of highly integrated, nonvolatile, memory devices: phase change random access memory (PRAM). Phase change random acess memory (PRAM) devices are synthesized using materials that undergo a reversible phase change between crystalline and amorphous phases, that have distinctly different resistances. The most commonly used phase change materials are ternary compositions of chalcogenide of group 14 and group 15 elements, such as germanium-antimony-tellurium compounds, commonly abbreviated as GST.

One of the technical hurdles in designing a PRAM cell is that in order to overcome the heat dissipation during the switching of GST materials from crystalline to amorphous states at certain temperatures, a high level of reset current has to be applied. This heat dissipation can be greatly reduced by confining the GST material into contact plugs, which in turn reduces the reset current needed. To build GST plugs on the substrate, atomic layer deposition (ALD) processes are used to produce films with high conformality and chemical composition uniformity.

Relevant prior art includes:
Sang-Wook Kim, S. Sujith, Bun Yeoul Lee, Chem. Commun., 2006, pp 4811-4813;
Stephan Schulz, Martin Nieger, J. Organometallic Chem., 570, 1998, pp 275-278;
Byung Joon Choi, et al. Chem Mater. 2007, 19, pp 4387-4389 ; Byung Joon Choi, et al. J. Electrochem. Soc., 154, pp H318-H324 (2007);
Ranyoung Kim, Hogi Kim, Soongil Yoon, Applied Phys. Letters, 89, pp 102-107 (2006);
Junghyun Lee, Sangjoon Choi, Changsoo Lee, Yoonho Kang, Daeil Kim, Applied Surface Science, 253 (2007) pp 3969-3976;
G. Becker, H. Freudenblum, O. Mundt, M. reti, M. Sachs, Synthetic Methods of Organometallic and Inorganic Chemistry, vol. 3, H.H. Karsch, New York, 1996, p. 193;
Sladek, A., Schmidbaur, H., Chem. Ber. 1995, 128, pp 565-567;
US patents and patent applications:
   US 2006/0049447 A1;
   US 2006/0039192 A1;
   US 2006/0072370 A1;
   US 2006/0172083 A1;
   US 8,148,197;
   US 2012/171812 A1; and
   US 7,817,464.

### BRIEF SUMMARY OF THE INVENTION

In one aspect, the present invention provides an ALD process for making an antimony-containing film on a surface of a substrate, the process comprising the steps of: introducing into a deposition chamber a germanium alkoxide as a precursor wherein the germanium alkoxide is represented by the formula Ge(OR¹⁴)₄, wherein R¹⁴ is a C₁-C₁₀ alkyl group or C₂-C₁₀ alkenyl group, a C₃-C₁₀ cyclic alkyl group, a C₃-C₁₀ cyclic alkenyl group, or a C₄-C₁₀ aromatic group, to form a molecular layer of germanium alkoxide on the surface of the substrate; and introducing into the deposition chamber a silylantimony precursor selected from the group consisting of: 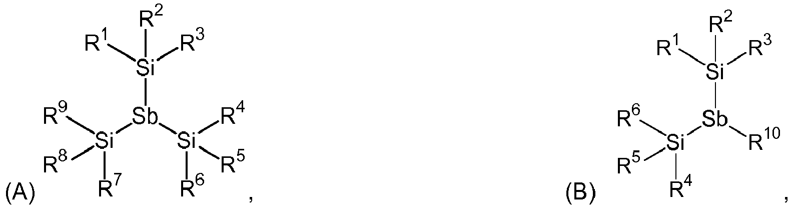 and 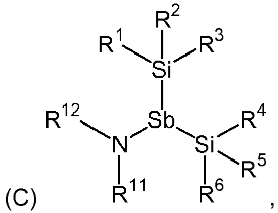 where R¹⁻¹⁰ are individually a hydrogen atom, a C₁-C₁₀ alkyl group or C₂-C₁₀ alkenyl group, a C₃-C₁₀ cyclic alkyl group, a C₃-C₁₀ cyclic alkenyl group, or a C₄-C₁₀ aromatic group; R¹¹ and R¹² are individually a C₁-C₁₀ alkyl group or C₃-C₁₀ alkenyl group, a C₃-C₁₀ cyclic alkyl group, a C₃-C₁₀ cyclic alkenyl group, or a C₄-C₁₀ aromatic group to form an Sb layer on top of the Te layer, wherein the Sb comprises silyl substituents.

In another aspect, the present invention provides an ALD process for making a germanium-bismuth-tellurium alloy film on a surface of a substrate, the process comprising the steps of: introducing into a deposition chamber a germanium alkoxide as a precursor wherein the germanium alkoxide is represented by the formula Ge(OR¹⁴)₄, wherein R¹⁴ is a C₁-C₁₀ alkyl group or C₂-C₁₀ alkenyl group, a C₃-C₁₀ cyclic alkyl group, a C₃-C₁₀ cyclic alkenyl group, or a C₄-C₁₀ aromatic group, to form a molecular layer of germanium alkoxide on the surface of the substrate; introducing into the deposition chamber a tellurium precursor selected from the group consisting of: 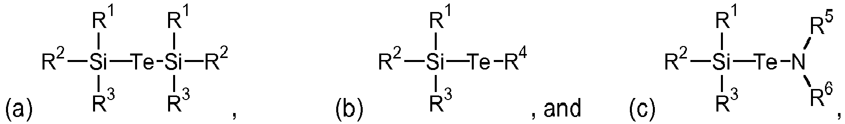 where R¹, R², R³, R⁴, R⁵, and R⁶ are independently hydrogen, a C₁-C₁₀ alkyl group or C₂-C₁₀ alkenyl group, a C₃-C₁₀ cyclic alkyl group, a C₃-C₁₀ cyclic alkenyl group, or a C₄-C₁₀ aromatic group to react with the germanium alkoxide layer to form a Te layer comprising Te-Ge bonds, wherein the Te comprises silyl substituents; reacting the silyl substituents on the Te to form Te-H bonds with (i) water and/or (ii) an alcohol having the general formula of ROH, where R is a C₁-C₁₀ alkyl group or C₂-C₁₀ alkenyl group, a C₃-C₁₀ cyclic alkyl group, a C₃-C₁₀ cyclic alkenyl group, or a C₄-C₁₀ aromatic group; introducing into the deposition chamber a silylantimony precursor selected from the group consisting of: 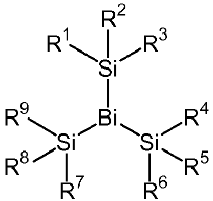 where R¹⁻¹⁰ are individually a hydrogen atom, a C₁-C₁₀ alkyl group or C₂-C₁₀ alkenyl group, a C₃-C₁₀ cyclic alkyl group, a C₃-C₁₀ cyclic alkenyl group, or a C₄-C₁₀ aromatic group to form an Sb layer on top of the Te layer, wherein the Bi comprises silyl substituents; and reacting the substituents on the Bi to form Bi-H bonds with (i) water and/or (ii) an alcohol having the general formula of ROH, where R is a C₁-C₁₀ alkyl group or C₂-C₁₀ alkenyl group, a C₃-C₁₀ cyclic alkyl group, a C₃-C₁₀ cyclic alkenyl group, or a C₄-C₁₀ aromatic group.

In yet another aspect, the present invention provides an ALD process for making an antimony- or bismuth-containing film on a surface of a substrate, the process comprising the steps of: Introducing ino a deposition chamber a silylantimony or bismuth precursor selected from the group consisting of: 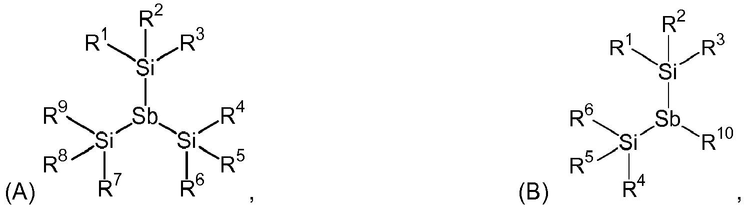 and 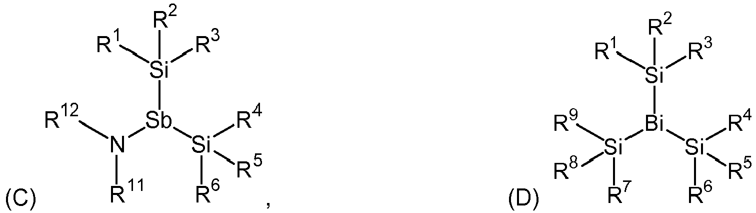 where R¹⁻¹⁰ are individually a hydrogen atom, an alkyl group or alkenyl group with 1 to 10 carbons as chain, branched, or cyclic, or an aromatic group; R¹¹ and R¹² are individually a C₁-C₁₀ alkyl group or C₃-C₁₀ alkenyl group, a C₃-C₁₀ cyclic alkyl group, a C₃-C₁₀ cyclic alkenyl group, or a C₄-C₁₀ aromatic group to form a silylantimony monolayer; and introducing into the deposition chamber a second precursor selected from the group consisting of:
M(OR¹³)₃, wherein M=Ga, In, Sb, and Bi; and R¹³ is a C₁-C₁₀ alkyl group or C₂-C₁₀ alkenyl group, a C₃-C₁₀ cyclic alkyl group, a C₃-C₁₀ cyclic alkenyl group, or a C₄-C₁₀ aromatic group,
M(OR¹³)₃₋ₓLₓ, wherein M=Sb or Bi; L is selected from Cl, Br, I, or mixtures thereof; x is 0, 1 or 2 with a proviso that x cannot be 0 when M=Sb ; and R¹³ is a C₁-C₁₀ alkyl group or C₂-C₁₀ alkenyl group, a C₃-C₁₀ cyclic alkyl group, a C₃-C₁₀ cyclic alkenyl group, or a C₄-C₁₀ aromatic group.

M(OR¹⁴)₄₋ₓLₓ, wherein M is selected from the group consisting of Ge, Sn, Pb; L is selected from Cl, Br, I, or mixtures thereof; x is 0, 1, 2 or 3; R¹⁴ is a C₁-C₁₀ alkyl group or C₂-C₁₀ alkenyl group, a C₃-C₁₀ cyclic alkyl group, a C₃-C₁₀ cyclic alkenyl group, or a C₄-C₁₀ aromatic group.

M(NR¹⁴R¹⁵)₃₋ₓLₓ wherein M is selected from the group consisting of Sb, Bi, Ga, In; L is selected from Cl, Br, I, or mixtures thereof; x is 1, 2 or 3; R¹⁴ is a C₁-C₁₀ alkyl group or C₃-C₁₀ alkenyl group, a C₃-C₁₀ cyclic alkyl group, a C₃-C₁₀ cyclic alkenyl group, or a C₄-C₁₀ aromatic group; and R¹⁵ is selected from the group consisting of hydrogen, a C₁-C₁₀ alkyl group or C₃-C₁₀ alkenyl group, a C₃-C₁₀ cyclic group, a C₃-C₁₀ cyclic alkenyl group, or a C₄-C₁₀ aromatic group, and

M(NR¹⁴R¹⁵)₄₋ₓLₓ wherein M is selected from the group consisting of Ge, Sn, Pb; L is selected from Cl, Br, I, or mixtures thereof; x is 1, 2 or 3; R¹⁴ is a C₁-C₁₀ alkyl group or C₃-C₁₀ alkenyl group, a C₃-C₁₀ cyclic alkyl group, a C₃-C₁₀ cyclic alkenyl group, or a C₄-C₁₀ aromatic group; and R¹⁵ is selected from the group consisting of hydrogen, a C₁-C₁₀ alkyl group or C₃-C₁₀ alkenyl group, a C₃-C₁₀ cyclic group, a C₃-C₁₀ cyclic alkenyl group, or a C₄-C₁₀ aromatic group.
Further aspects of the invention include:
#1. An ALD process for making an antimony-containing film on a surface of a substrate, the process comprising the steps of:
   introducing into a deposition chamber a germanium alkoxide as a precursor wherein the germanium alkoxide is represented by the formula Ge(OR¹⁴)₄, wherein R¹⁴ is a C₁-C₁₀ alkyl group, C₂-C₁₀ alkenyl group, a C₃-C₁₀ cyclic alkyl group, a C₃-C₁₀ cyclic alkenyl group, or a C₄-C₁₀ aromatic group, to form a molecular layer of germanium alkoxide on the surface of the substrate; and
   introducing into the deposition chamber a silylantimony precursor selected from the group consisting of: 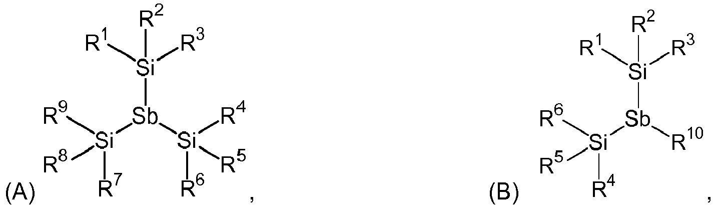 and 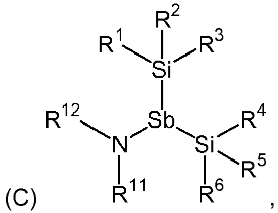 where R¹⁻¹⁰ are individually a hydrogen atom, a C₁-C₁₀ alkyl group, C₂-C₁₀ alkenyl group, a C₃-C₁₀ cyclic alkyl group, a C₃-C₁₀ cyclic alkenyl group, or a C₄-C₁₀ aromatic group; R¹¹ and R¹² are individually an a C₁-C₁₀ alkyl group or C₃-C₁₀ alkenyl group, a C₃-C₁₀ cyclic alkyl group, a C₃-C₁₀ cyclic alkenyl group, or a C₄-C₁₀ aromatic group to form an Sb layer on top of the Te layer, wherein the Sb comprises silyl substituents.
#2. The process of #1 wherein the silylantimony precursor is selected from the group consisting of tris(trimethylsilyl)antimony, tris(triethylsilyl)antimony, and tris(tertbutyldimethylsilyl)antimony, tris(dimethylsilyl)antimony.
#3. The process of #1 wherein the steps are repeated in sequence.
#4. The process of #1 wherein the temperature of the deposition chamber is between from room temperature to 400°C.
#5. An ALD process for making a germanium-bismuth-tellurium alloy film on a surface of a substrate, the process comprising the steps of:
   introducing into a deposition chamber a germanium alkoxide as a precursor wherein the germanium alkoxide is represented by the formula Ge(OR¹⁴)₄, wherein R¹⁴ is a C₁-C₁₀ alkyl group, C₂-C₁₀ alkenyl group, a C₃-C₁₀ cyclic alkyl group, a C₃-C₁₀ cyclic alkenyl group, or a C₄-C₁₀ aromatic group, to form a molecular layer of germanium alkoxide on the surface of the substrate;
   introducing into the deposition chamber a tellurium precursor selected from the group consisting of: 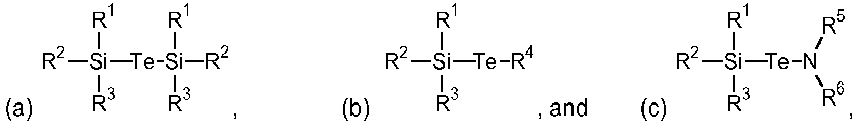 where R¹, R², R³, R⁴, R⁵, and R⁶ are independently hydrogen, a C₁-C₁₀ alkyl group or C₂-C₁₀ alkenyl group, a C₃-C₁₀ cyclic alkyl group, a C₃-C₁₀ cyclic alkenyl group, or a C₄-C₁₀ aromatic group to react with the germanium alkoxide layer to form a Te layer comprising Te-Ge bonds, wherein the Te comprises silyl substituents;
   reacting the silyl substituents on the Te to form Te-H bonds with (i) water and/or (ii) an alcohol having the general formula of ROH, where R is a C₁-C₁₀ alkyl group or C₂-C₁₀ alkenyl group, a C₃-C₁₀ cyclic alkyl group, a C₃-C₁₀ cyclic alkenyl group, or a C₄-C₁₀ aromatic group;
   introducing into the deposition chamber a silylantimony precursor selected from the group consisting of: 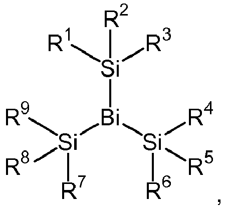 where R¹⁻¹⁰ are individually a hydrogen atom, a C₁-C₁₀ alkyl group or C₂-C₁₀ alkenyl group, a C₃-C₁₀ cyclic alkyl group, a C₃-C₁₀ cyclic alkenyl group, or a C₄-C₁₀ aromatic group to form an Sb layer on top of the Te layer, wherein the Bi comprises silyl substituents; and
   reacting the substituents on the Bi to form Bi-H bonds with (i) water and/or (ii) an alcohol having the general formula of ROH, where R is a C₁-C₁₀ alkyl group, C₂-C₁₀ alkenyl group, a C₃-C₁₀ cyclic alkyl group, a C₁-C₁₀ cyclic alkenyl group, or a C₄-C₁₀ aromatic group.
#6. The process of #5 wherein the silylbismuth precursor is selected from the group consisting of tris(trimethylsilyl)bismuth, tris(triethylsilyl)bismuth, tris(tertbutyldimethylsilyl)bismuth, and tris(dimethylsilyl)bismuth.
#7. The process of #5 wherein the steps are repeated in sequence.
#8. The process of #5 wherein the temperature of the deposition chamber is from room temperature to 400°C.
#9. The process of #5 wherein the alcohol is methanol.
#10. An ALD process for making an antimony- or bismuth-containing film on a surface of a substrate, the process comprising the steps of:
   Introducing ino a deposition chamber a silylantimony or bismuth precursor selected from the group consisting of: 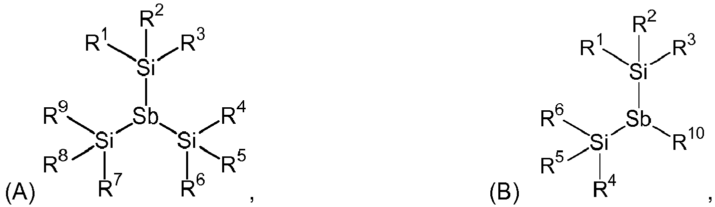 and 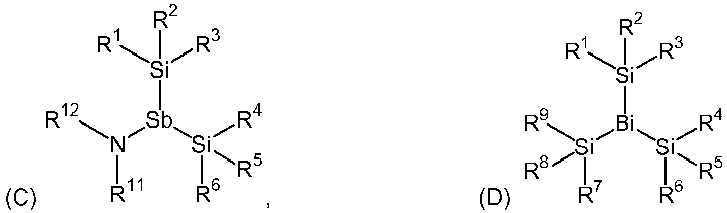 where R¹⁻¹⁰ are individually a hydrogen atom, an alkyl group or alkenyl group with 1 to 10 carbons as chain, branched, or cyclic, or an aromatic group; R¹¹ and R¹² are individually a C₁-C₁₀ alkyl group or C₃-C₁₀ alkenyl group, a C₃-C₁₀ cyclic alkyl group, a C₃-C₁₀ cyclic alkenyl group, or a C₄-C₁₀ aromatic group to form a silylantimony monolayer; and
   introducing into the deposition chamber a second precursor selected from the group consisting of:
      (a) M(OR¹³)₃, wherein M=Ga, In, Sb, and Bi; and R¹³ is a C₁-C₁₀ alkyl group, C₂-C₁₀ alkenyl group, a C₃-C₁₀ cyclic alkyl group, a C₃-C₁₀ cyclic alkenyl group, or a C₄-C₁₀ aromatic group,
      (b) M(OR¹³)₃₋ₓLₓ, wherein M=Sb or Bi; L is selected from Cl, Br, I, or mixtures thereof; x is 0, 1 or 2 with a proviso thatx cannot be 0 when M=Sb ; and R¹³ is a C₁-C₁₀ alkyl group or C₂-C₁₀ alkenyl group, a C₃-C₁₀ cyclic alkyl group, a C₃-C₁₀ cyclic alkenyl group, or a C₄-C₁₀ aromatic group,
      (c) M(OR¹⁴)₄₋ₓLₓ, wherein M is selected from the group consisting of Ge, Sn, Pb ; L is selected from Cl, Br, I, or mixtures thereof; x is 0, 1, 2 or 3; R¹⁴ is a C₁-C₁₀ alkyl group or C₂-C₁₀ alkenyl group, a C₃-C₁₀ cyclic alkyl group, a C₃-C₁₀ cyclic alkenyl group, or a C₄-C₁₀ aromatic group
      (d) M(NR¹⁴R¹⁵)₃₋ₓLₓ wherein M is selected from the group consisting of Sb, Bi, Ga, In; L is selected from Cl, Br, I, or mixtures thereof; x is 1, 2 or 3; R¹⁴ is a C₁-C₁₀ alkyl group or C₃-C₁₀ alkenyl group, a C₃-C₁₀ cyclic alkyl group, a C₃-C₁₀ cyclic alkenyl group, or a C₄-C₁₀ aromatic group; and R¹⁵ is selected from the group consisting of hydrogen, a C₁-C₁₀ alkyl group or C₃-C₁₀ alkenyl group, a C₃-C₁₀ cyclic group, a C₃-C₁₀ cyclic alkenyl group, or a C₄-C₁₀ aromatic group, and
      (e) M(NR¹⁴R¹⁵)₄₋ₓLₓ wherein M is selected from the group consisting of Ge, Sn, Pb; L is selected from Cl, Br, I, or mixtures thereof; x is 1, 2 or 3; R¹⁴ is a C₁-C₁₀ alkyl group or C₃-C₁₀ alkenyl group, a C₃-C₁₀ cyclic alkyl group, a C₃-C₁₀ cyclic alkenyl group, or a C₄-C₁₀ aromatic group; and R¹⁵ is selected from the group consisting of hydrogen, a C₁-C₁₀ alkyl group or C₃-C₁₀ alkenyl group, a C₃-C₁₀ cyclic group, a C₃-C₁₀ cyclic alkenyl group, or a C₄-C₁₀ aromatic group.
#11. The process of #6 wherein the silylantimony precursor is selected from the group consisting of tris(trimethylsilyl)antimony, tris(triethylsilyl)antimony, tris(tertbutyldimethylsilyl)antimony, and tris(dimethylsilyl)antimony.
#12. The process of #6 wherein the silylbismuth precursor is tris(trimethylsilyl)bismuth.
#13. The process of #6 wherein the steps are repeated in sequence.
#14. The process of #6 wherein the temperature of the deposition chamber is from room temperature to 400°C.
#15. The process of #6 wherein the second precursor is selected from the group consisting of SbCl(OMe)₂, SbCl₂(OMe), SbBr(OMe)₂, SbBr₂(OMe), Sbl(OMe)₂, SbCl(OEt)₂, SbCl₂(OEt), SbCl(OPrⁱ)₂, SbCl₂(OPrⁱ), BiCl(OMe)₂, BiCl₂(OMe), BiCl(OEt)₂, BiCl₂(OEt), BiCl(OPrⁱ)₂, BiCl₂(OPrⁱ), Cl₂SbNMeEt (II), Cl₂SbNEt₂ (III), CISb[NMe₂]₂ (IV), CISb[NMeEt]₂ (V), CISb[NEt₂]₂ (VI), Ga(NMe₂)₂Cl, Ga(NMe₂)Cl₂, [In(OCH₂CH₂NMe₂)₃]₂, [in(µ-O^{t}Bu)(O^{t}Bu)₂]₂, [In(OCMe₂Et)₂(m-OCMe₂Et)]₂, In[N(^{t}Bu)(SiMe₃)]₃, In(TMP)₃ (TMP=2,2,6,6-tetramethylpiperidino), and In(N(cyclohexyl)₂)₃.
#16. An ALD process for making an germanium-antimony-tellurium (GST) or germanium-bismuth-tellurium (GBT) film film on a surface of a substrate, the process comprising the steps of:
   Introducing into a deposition chamber a germainium precursor is selected from Ge(OR¹⁴)₄₋ₓLₓ, wherein L is selected from Cl, Br, I, or mixtures thereof; x is 0, 1, 2 or 3; R¹⁴ is a C₁-C₁₀ alkyl group, C₂-C₁₀ alkenyl group, a C₃-C₁₀ cyclic alkyl group, a C₃-C₁₀ cyclic alkenyl group, or a C₄-C₁₀ aromatic group;
   Introducing a silyltelluride precursor selected form the group consisting of The silyltellurium precursors can include disilyltellurium, silylalkyltellurium, or silylaminotellurium selected from the group consisting of: 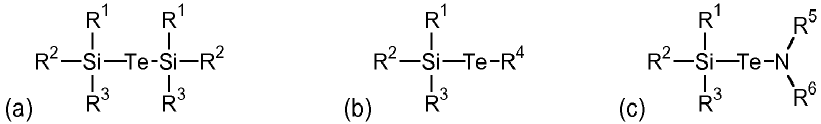
   where R¹, R², R³, R⁴, R⁵, and R⁶ are independently hydrogen, a C₁-C₁₀ alkyl group, C₂-C₁₀ alkenyl group, a C₃-C₁₀ cyclic alkyl group, a C₃-C₁₀ cyclic alkenyl group, or a C₄-C₁₀ aromatic group;
   Introducing into a deposition chamber a germainium precursor is selected from Ge(OR¹⁴)₄₋ₓLₓ, wherein L is selected from Cl, Br, I, or mixtures thereof; x is 0, 1, 2 or 3; R¹⁴ is a C₁-C₁₀ alkyl group or C₂-C₁₀ alkenyl group, a C₃-C₁₀ cyclic alkyl group, a C₃-C₁₀ cyclic alkenyl group, or a C₄-C₁₀ aromatic group;
   Introducing into a deposition chamber a silylantimony or silylbismuth precursor selected from the group consisting of: 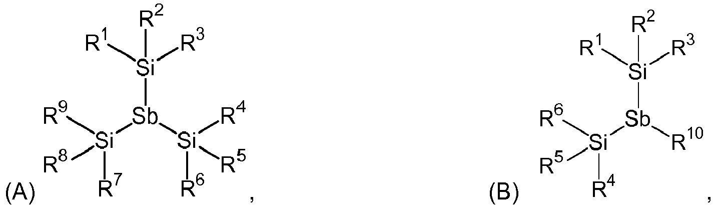 and 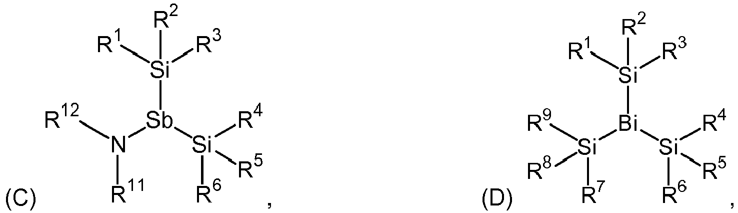
   where R¹⁻¹⁰ are individually a hydrogen atom, a C₁-C₁₀ alkyl group or C₂-C₁₀ alkenyl group, a C₃-C₁₀ cyclic alkyl group, a C₃-C₁₀ cyclic alkenyl group, or a C₄-C₁₀ aromatic group; R¹¹ and R¹² are individually a C₁-C₁₀ alkyl group or C₃-C₁₀ alkenyl group, a C₃-C₁₀ cyclic alkyl group, a C₃-C₁₀ cyclic alkenyl group, or a C₄-C₁₀ aromatic group to form a silylantimony or silylbismuth monolayer; and
   repeating the steps above until a desired thickness is reached.
#17. The process of #11 wherein the germainium precursor is selected from the group consisting of Ge(OMe)₄, Ge(OEt)₄, Ge(OPrⁿ)₄, Ge(OPrⁱ)₄, GeCl(OMe)₃, GeCl₂(OMe)₂, GeCl₃(OMe), GeCl(OEt)₃, GeCl₂(OEt)₂, GeCl₃(OEt), GeCl(OPrⁿ)₃, GeCl(OPrⁿ)₃, GeCl₂( OPrⁿ))₂, GeCl₂(OPrⁱ)₂, GeCl₃(OPrⁱ), GeCl(OBu^{t})₃, GeCl₂(OBu^{t}))₂, and GeCl₃(OBu^{t}).
#18. The process of #11 wherein the silylantimony precursor is selected from the group consisting of tris(trimethylsilyl)antimony, tris(triethylsilyl)antimony, tris(tertbutyldimethylsilyl)antimony, and tris(dimethylsilyl)antimony.
#19. The process of #11 wherein the silylbismuth precursor is selected from the group consisting of tris(trimethylsilyl)bismuth, tris(triethylsilyl)bismuth, and tris(tertbutyldimethylsilyl)bismuth.
#20. The process of #11 wherein the disilyltellurium precursor is selected from the group consisting of bis(trimethylsilyl)tellurium, bis(triethylsilyl)tellurium, and bis(tertbutyldimethylsilyl)tellurium.
   In a further aspect the invention relates to a precursor as described herein. Preferably, the precursor is a silylantimony precursor, a silylbismuth precursor, or a silyltellurium precursor.
   Features described in connection with one aspect of the invention can be used in connection with any other aspect of the invention.

### DETAILED DESCRIPTION OF THE INVENTION

The present invention provides a class of antimony or bismuth precursors, which generate antimony and/or bismuth layers in an ALD process. The antimony or bismuth or antimony-bismuth alloy layer may react with subsequently deposited germanium and tellurium layers in ALD cycles to form GST ternary material films, which are suitable for PRAM devices.

GST or GBT (germanium-bismuth-tellurium) materials in PRAM devices are normally deposited in the temperature range of 180° - 300° C. It was found that films deposited at 200° C have the best chemical and structural properties. The ALD process requires precursors with high chemical reactivity and reaction selectivity. Currently existing precursors, such as dialkyltellurium, trialkylantimony, and alkylgermanes do not have the required reactivity at given deposition conditions to be used in ALD cycles. Frequently, plasma is used to promote the deposition.

This invention provides silylantimony compounds as ALD precursors, which may react with alcohols or water to generate an antimony layer. With subsequent deposition of germanium and tellurium e.g. from tetraaminogermanium and organotellurium precursors, a GST or GBT film can be deposited on a substrate with high conformality.

The present invention provides silylantimony or silylbismuth precursors, which generate antimony or bismuth layers in an ALD process. The antimony or bismuth layer may react with subsequently deposited germanium and tellurium layers in a plurality of ALD cycles to form GST or GBT ternary material films, which are suitable for PRAM devices. In certain embodiments, this invention discloses several silylantimony precursors with high reactivity and thermal stability, and the chemistries to be used in an ALD process to deposit a GST or GBT film in conjunction with other chemicals.

In other embodiments, this invention provides silylantimony or silylbismuth compounds as ALD precursors, which react with alcohols or water to generate antimony atomic layers. With consequent deposition of germanium and tellurium from tetraaminogermanium and tellurium precursor, GST or GBT films can be deposited on a substrate with high conformality.

In certain embodiments, the antimony or bismuth precursors include trisilylantimony, disilylalkylantimony, disilylantimony, disilylaminoantimony or trisilylbismuth selected from: 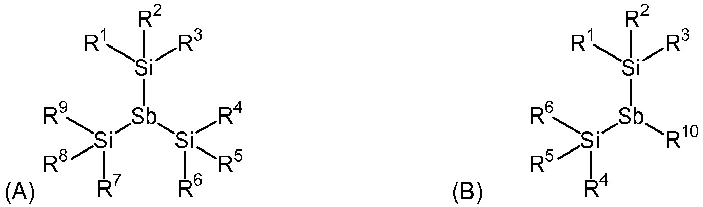 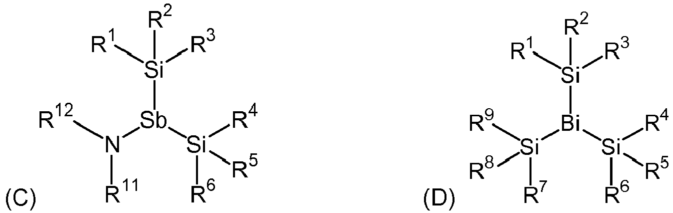 where R¹⁻¹⁰ are individually a hydrogen atom, an alkyl group or alkenyl group with 1 to 10 carbons in linear, branched, or cyclic form, or an aromatic group; and R¹¹ and R¹² are individually a C₁-C₁₀ alkyl group, a C₂-C₁₀ alkenyl (preferably C₃-C₁₀ alkenyl) group, a C₃-C₁₀ cyclic alkyl group, a C₃-C₁₀ cyclic alkenyl group, or a C₄-C₁₀ aromatic group. In certain embodiments, R¹ is a hydrogen atom, a C₁-C₁₀ alkyl group;C₂-C₁₀ alkenyl group, a C₃-C₁₀ cyclic alkyl group, a C₃-C₁₀ cyclic alkenyl group, or a C₄-C₁₀ aromatic group. Preferably, if in structure (A) one of R¹⁻⁹ is aromatic, then the remaining R¹⁻⁹ groups on the silicon bearing the aromatic group are not both methyl. Preferably, if in structure (A) any of R¹⁻⁹ are C¹⁻³ or phenyl, then not all of R¹⁻⁹ are the same.

Throughout the description, the term "alkyl" denotes a linear or branched functional group having from 1 to 10 or 1 to 6 carbon atoms. Exemplary alkyl groups include, but are not limited to, methyl, ethyl, propyl, isopropyl, butyl, isobutyl, sec-butyl, tert-butyl, pentyl, iso-pentyl, tert-pentyl, hexyl, iso-hexyl, and neo-hexyl. In certain embodiments, the alkyl group may have one or more functional groups such as, but not limited to, an alkoxy group, a dialkylamino group or combinations thereof, attached thereto. In other embodiments, the alkyl group does not have one or more functional groups attached thereto. The term "cyclic alkyl" denotes a cyclic functional group having from 3 to 10 or from 4 to 10 carbon atoms or from 5 to 10 carbon atoms. Exemplary cyclic alkyl groups include, but are not limited to, cyclobutyl, cyclopentyl, cyclohexyl, and cyclooctyl groups. The term "aromatic" denotes an aromatic cyclic functional group having from 4 to 10 carbon atoms or from 6 to 10 carbon atoms. Exemplary aryl groups include, but are not limited to, phenyl, benzyl, chlorobenzyl, tolyl, and o-xylyl. The term "alkenyl group" denotes a group which has one or more carbon-carbon double bonds and has from 2 to 10 or from 2 to 6 or from 2 to 4 carbon atoms.

Exemplary trisilylantimony or trisilylbimuth precursors include, for example, tri(trimethylsilyl)antimony, tri(triethylsilyl)antimony, and tri(tert-butyldimethylsilyl)antimony, tri(trimethylsilyl)bismuth, tri(triethylsilyl)bismuth, and tri(tert-butyldimethylsilyl)bismuth, tris(dimethylsilyl)antimony.

Silylantimony or silylbismuth compounds are highly reactive with alcohols or water. The reaction generates elemental antimony or bismuth at low temperature:

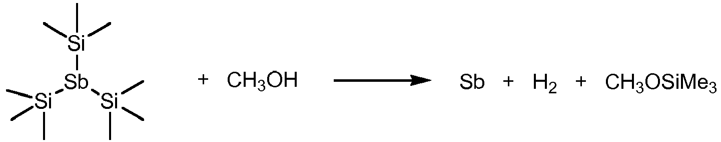

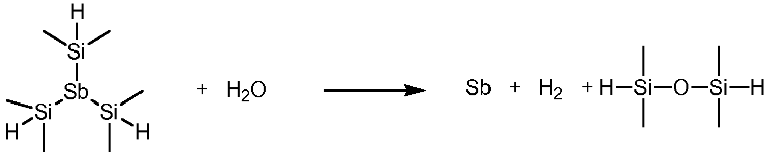

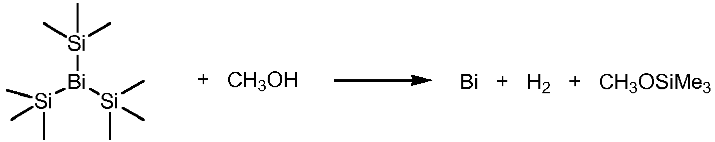

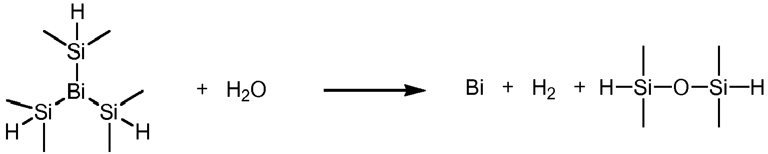

In other embodiments of the present invention, metallic antimony or antimony alloy can be deposited by reacting silylantimony or silylbismuth compounds with metal compound alkoxides and/or mixed halide and alkoxide compounds. A metal alkoxide includes compounds represented by the formula M(OR¹³)₃, wherein M=Ga, In, Sb or Bi; R¹³ is a C₁-C₁₀ alkyl group, aC₂-C₁₀ alkenyl group, a C₃-C₁₀ cyclic alkyl group, a C₃-C₁₀ cyclic alkenyl group, or a C₄-C₁₀ aromatic group. A mixed halide and alkoxide metal compound includes compounds represented by the formula M(OR¹³)₃₋ₓLₓ, wherein M=Ga, In, Sbor Bi; L is selected from Cl, Br, I, or mixtures thereof; x is 1 or 2; and R¹³ is as defined above. Example of such compounds include, for example, SbCl(OMe)₂, SbCl₂(OMe), SbBr(OMe)₂, SbBr₂(OMe), Sbl(OMe)₂, SbCl(OEt)₂, SbCl₂(OEt), SbCl(OPrⁱ)₂, SbCl₂(OPrⁱ), BiCl(OMe)₂, BiCl₂(OMe), BiCl(OEt)₂, BiCl₂(OEt), BiCl(OPrⁱ)₂, BiCl₂(OPrⁱ).

These reactions can take place at temperatures in the range of room temperature to 400° C as demonstrated below.

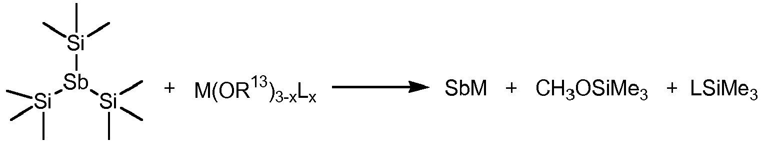

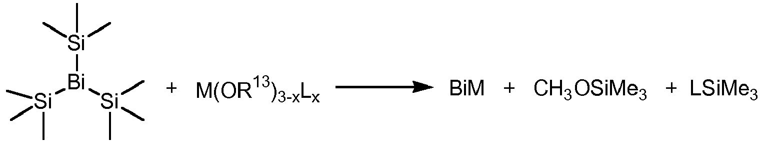

In an ALD process, the silylantimony precursors, alcohols, germanium and tellurium precursors, such as Ge(OMe)₄ and (Me₃Si)₂Te (wherein "Me" is methyl) may be introduced to a deposition chamber in a cyclic manner by vapor draw or direct liquid injection (DLI). The deposition temperature is preferably between room temperature and 400°C.

The ALD reaction to deposit GBT films can be illustrated by the following scheme: 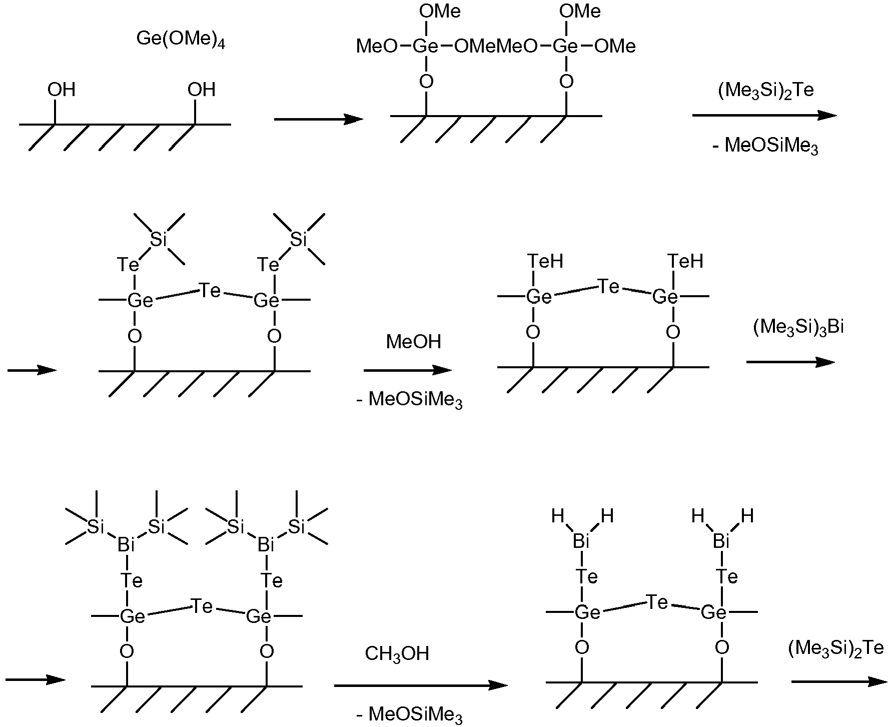
Step 1. Tetrakis(methoxy)germane is introduced and forms a molecular layer of alkoxygermane on the surface of the substrate.
Step 2. Hexamethyldisilyltellurium reacts with alkoxygermane layer to form Te-Ge bonds with elimination of methoxytrimethylsilane. A Te layer with silyl substituents is formed.
Step 3. Methanol reacts with remaining silyl groups on the tellurium layer to form Te-H bonds and a volatile byproduct, methoxytrimethylsilane, which is removed by purge.
Step 4. Tris(trimethylsilyl)bismuth is introduced and forms a bismuth layer on the top of the tellurium layer.
Step 5. Methanol reacts with the remaining silyl groups on the antimony layer to form Bi-H bonds and a volatile byproduct, methoxytrimethylsilane, which is removed by purge.
Step 6. Hexamethyldisilyltellurium is introduced again and forms a tellurium layer.
Step 7. Methanol is introduced again to remove silyl groups on the tellurium.

Another ALD reaction can be illustrated by the following two schemes for deposting Ge-Te-Ge-Sb (first scheme) or Ge-Te-Ge-Bi (second scheme) films: 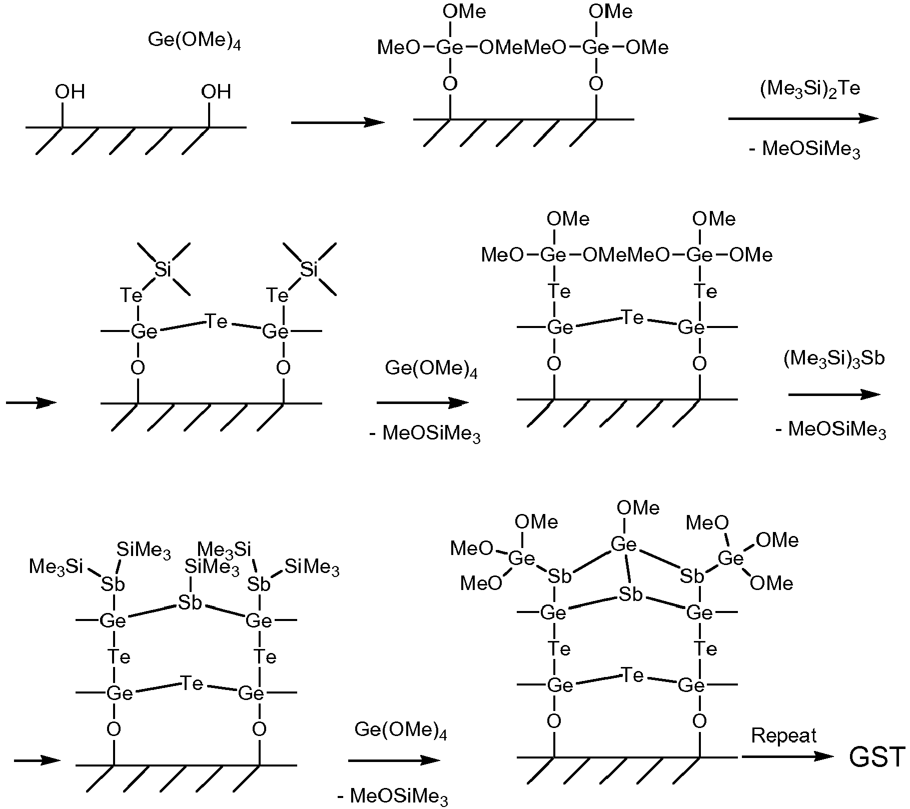 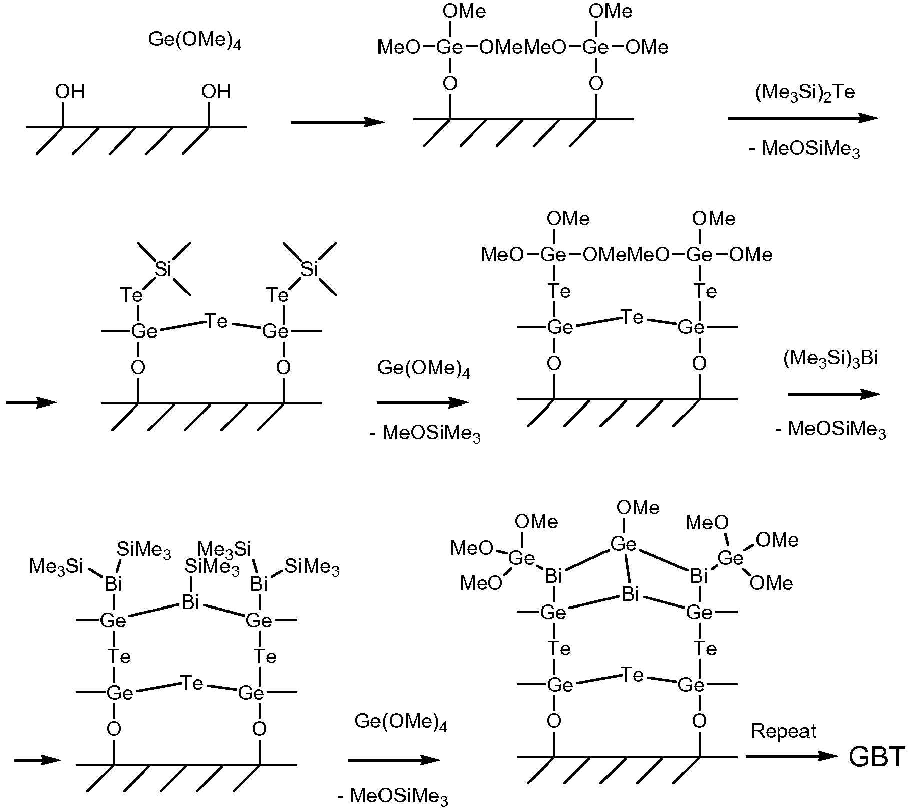
Step 1. Tetrakis(methoxy)germane is introduced and forms a molecular layer of alkoxygermane on the surface of the substrate.
Step 2. Hexamethyldisilyltellurium reacts with alkoxygermane layer to form Te-Ge bonds with elimination of methoxytrimethylsilane. A Te layer with silyl substituents is formed.
Step 3. Tetrakis(methoxy)germane reacts with remaining silyl groups on the layer to form Te-Ge bonds with elimination of methoxytrimethylsilane. A Ge layer with methoxy substituents is formed.
Step 4. Tris(trimethylsilyl)antimony or tris(trimethylsilyl)bismuth is introduced to form an antimony or bismuth layer with silyl substituents on the top of the germanium layer via elimination of methoxytrimethylsilane.
Step 5. Tetrakis(methoxy)germane reacts with remaining silyl groups on the Sb or Bi layer to form Sb-Ge or Bi-Ge bonds, generating a Ge layer with methoxy substituents.

An ALD cycle is then repeated, potentially many times, until the desired film thickness is achieved. The next cycle starts with step 1, again, etc. In another embodiment, step 2 and step 4 can be switched, e.g. if Ge-Sb-Ge-Te or Ge-Bi-Ge-Te films are to be deposited.

In certain embodiments, the silylantimony or silylbismuth compounds used in this process are selected from (A), (B), (C) and (D) defined above.

Alkoxygermanes used in this process preferably have the general formula: 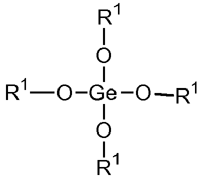 where R¹ is a hydrogen atom, a C₁-C₁₀ alkyl group, a C₂-C₁₀ alkenyl group, a C₃-C₁₀ cyclic alkyl group, a C₃-C₁₀ cyclic alkenyl group, or a C₄-C₁₀ aromatic group.

In yet another embodiment of the present invention, GST films can be formed by employing a germanium compound as a precursor wherein the germanium compound optionally having both halide and alkoxy ligand is represented by the formula Ge(OR¹⁴)₄₋ₓLₓ, wherein L is selected from Cl, Br, I, or mixtures thereof; x is 0, 1, 2 or 3; R¹⁴ is a C₁-C₁₀ alkyl group, a C₂-C₁₀ alkenyl group, a C₃-C₁₀ cyclic alkyl group, a C₃-C₁₀ cyclic alkenyl group, or a C₄-C₁₀ aromatic group. The germanium compound precursor can be reacted with, for example, silylantimony, silylbismuth, or silyltellurium in the same manner as M(OR¹³)₃₋ₓLₓ as described above. Examples of germanium compound having both halide and alkoxy ligand include, for example, GeCl(OMe)₃, GeCl₂(OMe)₂, GeCl₃(OMe), GeCl(OEt)₃, GeCl₂(OEt)₂, GeCl₃(OEt), GeCl(OPrⁿ)₃, GeCl₂(OPrⁿ))₂, GeCl₃(OPrⁿ), GeCl(OPrⁱ)₃, GeCl₂(OPrⁱ))₂, GeCl₃(OPrⁱ), GeCl(OBu^{t})₃, GeCl₂(OBu^{t}))₂, and GeCl₃(OBu^{t}), wherein OBu^{t} is tert-butyl alkoxy, OPrⁿ is n-propoxy , and OPrⁱ is iso-propoxy. Such compounds are preferably thermally stable and have bulky alkoxy groups which prevents disproportionation reactions.

The silyltellurium precursors can include disilyltellurium, silylalkyltellurium, or silylaminotellurium selected from: 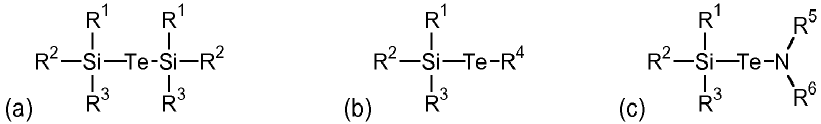 where R¹, R², R³, R⁴, R⁵, and R⁶ are independently hydrogen, a C₁-C₁₀ alkyl group, aC₂-C₁₀ alkenyl group, a C₃-C₁₀ cyclic alkyl group, a C₃-C₁₀ cyclic alkenyl group, or a C₄-C₁₀ aromatic group. Exemplary disilytellurium precursors include, for example, bis(trimethylsilyl)tellurium, bis(triethylsilyl)tellurium, and bis(tertbutyldimethylsilyl)tellurium.

In other embodiments of the present invention, antimony or bismuth-containing films can be made by reacting a silylantimony or silylbismuth compound with mixed amino and halide compounds with a formula of M(NR¹⁴R¹⁵)₃₋ₓLₓ or M(NR¹⁴R¹⁵)₄₋ₓLₓ wherein M is selected from Sb, Bi, Ga, In, Ge, Sn, Pb; L is selected from Cl, Br, I, or mixtures thereof; x is 1, 2 or 3; R¹⁴ is a C₁-C₁₀ alkyl group, a C₂-C₁₀ alkenyl group (preferably C₃-C₁₀ alkenyl), a C₃-C₁₀ cyclic alkyl group, a C₃-C₁₀ cyclic alkenyl group, or a C₄-C₁₀ aromatic group; and R¹⁵ is selected from hydrogen, a C₁-C₁₀ alkyl group, a C₂-C₁₀ alkenyl group (preferably C₃-C₁₀ alkenyl), a C₃-C₁₀ cyclic group, a C₃-C₁₀ cyclic alkenyl group, or a C₄-C₁₀ aromatic group.

For example, germanium compounds having both amino and halide ligands that are suitable for use in the process of the present invention are described in V. N. Khrustalev et al. "New Stable Germylenes, Stannylenes, and related compounds. 8. Amidogermanium(II) and -tin(II) chlorides R2NE14Cl (E14 = Ge, R=Et; Sn, R=Me) Revealing New Structural Motifs", Appl. Organometal. Chem., 2007; 21: 551-556. An example of such compounds is [GeCl(NMe₂)]₂.

Antimony compounds having mixed amino and halide ligands suitable for use in the process of the present invention include those disclosed in Ensinger, U. and A. Schmidt (1984), "Dialkylaminostibines. Preparation and spectra" Z. Anorg. Allg. Chem. FIELD Full Journal Title:Zeitschrift fuer Anorganische und Allgemeine Chemie 514: 137-48; and Ensinger, U., W. Schwarz, B. Schrutz, K. Sommer and A. Schmidt (1987) "Methoxostibines. Structure and vibrational spectra." Z. Anorg. Allg. Chem. FIELD Full Journal Title:Zeitschrift fuer Anorganische und Allgemeine Chemie 544: 181-91. Examples of such compounds include, for example, Cl₂SbNMe₂ (I), Cl₂SbNMeEt (II), Cl₂SbNEt₂ (III), ClSb[NMe₂]₂ (IV), ClSb[NMeEt]₂ (V), ClSb[NEt₂]₂ (VI), Ga(NMe₂)₂Cl, and Ga(NMe₂)Cl₂.

Indium compounds suitable for use in the process of the present invention include those disclosed by Frey, R., V. D. Gupta and G. Linti (1996). "Monomeric bis and tris(amides) of indium" 622(6): 1060-1064; Carmalt, C. J. and S. J. King (2006). "Gallium(III) and indium(III) alkoxides and aryloxides." Coordination Chemistry Reviews 250(5-6): 682-709; Carmalt, C. J. (2001). "Amido compounds of gallium and indium." Coordination Chemistry Reviews 223(1): 217-264; Frey, R., V. D. Gupta and G. Linti (1996). "Monomeric bis and tris(amides) of indium." Monomere bis- und tris(amide) des indiums 622(6): 1060-1064; Suh, S. and D. M. Hoffman (2000). "General Synthesis of Homoleptic Indium Alkoxide Complexes and the Chemical Vapor Deposition of Indium Oxide Films." Journal of the American Chemical Society 122(39): 9396-9404. Examples of such compounds include, for example, [In(OCH₂CH₂NMe₂)₃]₂, [In(µ-O^{t}Bu)(O^{t}Bu)₂]₂, [In(OCMe₂Et)₂(µ-OCMe₂Et)]₂, In[N(^{t}Bu)(SiMe₃)]₃, In(TMP)₃ (TMP=2,2,6,6-tetramethylpiperidino), and In(N(cyclohexyl)₂)₃.

Alcohols used in this process have the general formula:

ROH

where R is an alkyl group or alkenyl group with 1 to 10 carbons in linear, branched, or cyclic form or an aromatic group. For example, R can be a C₁-C₁₀ alkyl group, a C₂-C₁₀ alkenyl group, a C₃-C₁₀ cyclic alkyl group, a C₂-C₁₀ cyclic alkenyl group, or a C₄-C₁₀ aromatic group. In certain embodiments, methanol is preferred.

### EXAMPLES

### Example 1: Synthesis of Tris(trimethylsilyl)antimony

1.22g (0.01 mol) of 200 mesh antimony powder, 0.72 g (0.03 mol) of lithium hydride, and 40 ml of tetrahydrofuran (THF) were placed in a 100 ml flask. With stirring, the mixture was refluxed for 4 hours. All of the black powder constituting antimony disappeared, and a muddy colored precipitate was formed. Then, the mixture was cooled down to - 20 °C; 3.3 g (0.03 mol) of trimethylchlorosilane was added. The mixture was allowed to warm up to room temperature. After stirring for 4 hours, the mixture was filtered under inert atmosphere. The solvent was removed by distillation. Tris(trimethylsilyl)antimony was purified by vacuum distillation.

### Example 2: Synthesis of Tris(dimethylsilyl)antimony

1.22g (0.01 mol) of 200 mesh antimony powder, 0.72 g (0.03 mol) of lithium hydride, and 40 ml of tetrahydrofuran (THF) were placed in a 100 ml flask. With stirring, the mixture was refluxed for 4 hours. All of the black powder constituting antimony disappeared, and a muddy colored precipitate was formed. Then, the mixture was cooled down to - 20 °C; 2.83 g (0.03 mol) of dimethylchlorosilane was added. The mixture was allowed to warm up to room temperature. After stirring for 4 hours, the mixture was filtered under inert atmosphere. The solvent was removed by distillation. Tris(dimethylsilyl)antimony was purified by vacuum distillation.

### Example 3: Synthesis of Tris(dimethylsilyl)antimony

3.65 g (0.03 mol) of 200 mesh antimony powder, 2.07 g (0.09 mol) of sodium, 1.15g (0.009 mol) of naphthalene, and 50 ml of THF were placed in a 100 ml flask. The mixture was stirred at room temperature for 24 hours. All of the black powder constituting antimony and sodium disappeared, and a muddy colored precipitate was formed. Then, the mixture was cooled down to - 20°C; 8.51 g (0.09 mol) of dimethylchlorosilane was added. The mixture was allowed to warm up to room temperature. After stirring for 4 hours, the mixture was filtered under inert atmosphere. The solvent was removed by distillation. Tris(dimethylsilyl)antimony was purified by vacuum distillation.

### Example 4: Synthesis of Tris(trimethylsilyl)bismuth (Prophetic)

6.27 g (0.03 mol) of 200 mesh bismuth powder, 2.07 g (0.09 mol) of sodium, 1.15g (0.009 mol) of naphthalene, and 50 ml of THF is placed in a 100 ml flask. The mixture is stirred at room temperature for 24 hours. All of the black powder constituting bismuth and sodium disappears, and a muddy colored precipitate forms. Then, the mixture is cooled down to - 20 °C; 9.77 g (0.09 mol) of trimethylchlorosilane is added. The mixture is allowed to warm up to room temperature. After stirring for 4 hours, the mixture is filtered under inert atmosphere. The solvent is removed by distillation. Tris(trmethylsilyl)bismuth can be purified by vacuum distillation.

### Example 5: Generation of Antimony Film

0.05 g of tris(dimethylsilyl)antimony was placed on the bottom of a 100 ml pyrex glass flask filled with nitrogen and fitted with a rubber septum. 0.1 g of methanol was added slowly with a syringe. A shiny black film started to deposit inside the glass wall of the flask. After a few minutes, the entire flask interior was coated with a dark gray / black antimony film.

### Example 6: Synthesis of Germanium Bismuthide (Prophetic)

0.43 g (0.001 mol) tris(trimethylsilyl)bismuth is dissolved in 6 ml of acetonitrile. To the solution, 0.12 g tetramethoxygermane is added at room temperature. The reaction is exothermic. A black precipitate forms immediately. The precipitate is filtered out and washed with THF, and dried in air. Energy Dispersive X-ray Analysis (EDX) in conjunction with Scanning Electron Microscopy (SEM) can be used to study the black solid precipitate. The results will indicate that the black solid is a composition of germanium and bismuth. Germanium bismuthide is insoluble in organic solvents.

### Example 7: Synthesis of Indium Antimonide (Prophetic)

0.38 g (0.001 mol) indium tri-t-pentoxide is dissolved in 6 ml of acetonitrile. To the solution, 0.34 g (0.001 mol) Tris(trimethylsilyl)antimony is added at room temperature. The reaction is exo-thermic. A black precipitate is formed immediately. The precipitate is filtered out and washed with THF, and dried in air. Energy Dispersive X-ray Analysis (EDX) in conjunction with Scanning Electron Microscopy (SEM) can be used to study the black solid precipitate. The results will indicate that the black solid is a composition of indium and antimony. Indium antimonide is insoluble in organic solvents.

### Example 8: Synthesis of Bismuth Antimonide (Prophetic)

0.34 g (0.001 mol) bismuth triethoxide is dissolved in 6 ml of acetonitrile. To the solution, 0.34 g (0.001 mol) Tris(trimethylsilyl)antimony is added at room temperature. The reaction is exo-thermic. A black precipitate is formed immediately. The precipitate is filtered out and washed with THF, and dried in air. Energy Dispersive X-ray Analysis (EDX) in conjunction with Scanning Electron Microscopy (SEM) can be used to study the black solid precipitate. The results indicated that the black solid is a composition of antimony and bismuth. Bismuth antimonide is insoluble in organic solvents.

### Example 9: Deposition of GeBi Films in ALD Reactor (Prophetic)

Deposition of GeBi film using atomic layer deposition (ALD) technique including the following steps:
a) Substrates on which films are to be deposited are loaded to an ALD reactor;
b) The reactor is flashed with N₂ and pumped down to low pressure of less than 1 torr (130 Pa) and heated up to a temperature at which film deposition is performed;
c) A fixed flow rate of the vapor of silylbismuth compound as Bi precursor is introduced to the reactor. The reactor is saturated with this vapor for a short fixed time (typical less than 5 seconds), and then pumped down to 1 torr (130 Pa), followed by flashing with N₂;
d) A fixed flow rate of the vapor of alkoxygermane compound as Ge precursor is introduced to the reactor. The reactor is saturated with this vapor for a short fixed time (typical less than 5 seconds), and then pumped down to 1 torr (130 Pa), followed by flashing with N₂; and
steps c) to d) are repeated until a desired thickness of the film is achieved. In another example, alkoxygermane compound can be introduced in step c) while silylbismuth compound is introduced in step d).

With the deposition chemistry, highly conformal GeBi films can be deposited on the surface of substrate materials such as silicon, silicon oxide, silicon nitride, titanium nitride. The process temperature range could be from room temperature to 400°C.

### Example 10: Deposition of Sb Films in ALD Reactor

Deposition of antimony film using atomic layer deposition (ALD) technique including the following steps:
a) Substrates on which films are to be deposited are loaded to an ALD reactor;
b) The reactor is flashed with N₂ and pumped down to low pressure of less than 1 torr (130 Pa) and heated up to a temperature at which film deposition is performed;
c) A fixed flow rate of the vapor of trisilylantimony compound is introduced to the reactor. The reactor is saturated with this vapor for a short fixed time (typical less than 5 seconds), and then pumped down to 1 torr (130 Pa), followed by flashing with N₂;
d) A fixed flow rate of the vapor of alkoxyantimony compound is introduced to the reactor. The reactor is saturated with this vapor for a short fixed time (typical less than 5 seconds), and then pumped down to 1 torr (130 Pa), followed by flashing with N₂; and
steps c) to d) are repeated until a desired thickness of the film is achieved. In another example, alkoxyantimony compound can be introduced in step c) while trisilylantimony compound is introduced in step d).
With the deposition chemistry, highly conformal antimony films can be deposited on the surface of substrate materials such as silicon, silicon oxide, silicon nitride, titanium nitride. The process temperature range could be from room temperature to 400°C.

### Example 11: Deposition of GeSbTe Films in ALD Reactor

Deposition of GeSbTe film using atomic layer deposition (ALD) technique including the following steps:
a) Substrates on which films are to be deposited are loaded to an ALD reactor;
b) The reactor is flashed with N₂ and pumped down to low pressure of less than 1 torr (130 Pa) and heated up to a temperature at which film deposition is performed;
c) A fixed flow rate of the vapor of alkoxygermane compound as Ge precursor is introduced to the reactor. The reactor is saturated with this vapor for a short fixed time (typical less than 5 seconds), and then pumped down to 1 torr (130 Pa), followed by flashing with N₂; and
d) A fixed flow rate of the vapor of disilyltellurium compound as Te precursor is introduced to the reactor. The reactor is saturated with this vapor for a short fixed time (typical less than 5 seconds), and then pumped down to 1 torr (130 Pa), followed by flashing with N₂;
e) A fixed flow rate of the vapor of alkoxygermane compound as Ge precursor is introduced to the reactor. The reactor is saturated with this vapor for a short fixed time (typical less than 5 seconds), and then pumped down to 1 torr (130 Pa), followed by flashing with N₂;
f) A fixed flow rate of the vapor of trisilylantimony compound as Sb precursor is introduced to the reactor. The reactor is saturated with this vapor for a short fixed time (typical less than 5 seconds), and then pumped down to 1 torr (130 Pa), followed by flashing with N₂; and
steps c) to f) are repeated until a desired thickness of the film is achieved.
With the deposition chemistry, highly conformal GeSbTe films can be deposited on the surface of substrate materials such as silicon, silicon oxide, silicon nitride, titanium nitride. The process temperature range could be from room temperature to 400°C.

### Example 12: Deposition of GeBiTe Films in ALD Reactor (prophetic)

Deposition of GeBiTe film using atomic layer deposition (ALD) technique including the following steps:
a) Substrates on which films are to be deposited are loaded to an ALD reactor;
b) The reactor is flashed with N₂ and pumped down to low pressure of less than 1 torr (130 Pa) and heated up to a temperature at which film deposition is performed;
c) A fixed flow rate of the vapor of alkoxygermane compound as Ge precursor is introduced to the reactor. The reactor is saturated with this vapor for a short fixed time (typical less than 5 seconds), and then pumped down to 1 torr (130 Pa), followed by flashing with N₂; and
d) A fixed flow rate of the vapor of disilyltellurium compound as Te precursor is introduced to the reactor. The reactor is saturated with this vapor for a short fixed time (typical less than 5 seconds), and then pumped down to 1 torr (130 Pa), followed by flashing with N₂;
e) A fixed flow rate of the vapor of alkoxygermane compound as Ge precursor is introduced to the reactor. The reactor is saturated with this vapor for a short fixed time (typical less than 5 seconds), and then pumped down to 1 torr (130 Pa), followed by flashing with N₂;
f) A fixed flow rate of the vapor of trisilylbismuth compound as Bi precursor is introduced to the reactor. The reactor is saturated with this vapor for a short fixed time (typical less than 5 seconds), and then pumped down to 1 torr (130 Pa), followed by flashing with N₂; and
steps c) to f) are repeated until a desired thickness of the film is achieved.

With the deposition chemistry, highly conformal GeBiTe films can be deposited on the surface of substrate materials such as silicon, silicon oxide, silicon nitride, titanium nitride. The process temperature range could be from room temperature to 400°C.

Further aspects of the invention include:
#1. An ALD or CVD process for making a germanium-bismuth-tellurium alloy film on a surface of a substrate, the process comprising the steps of:
   introducing into a deposition chamber a germanium alkoxide as a precursor wherein the germanium alkoxide is represented by the formula Ge(OR¹⁴)₄, wherein R¹⁴ is selected from a C₁-C₁₀ alkyl group, a C₂-C₁₀ alkenyl group, a C₃-C₁₀ cyclic alkyl group, a C₃-C₁₀ cyclic alkenyl group, or a C₄-C₁₀ aromatic group, to form a molecular layer of germanium alkoxide on the surface of the substrate;
   introducing into the deposition chamber a tellurium precursor selected from (a), (b) and (c), to react with the germanium alkoxide layer to form a Te layer comprising Te-Ge bonds, wherein the Te comprises silyl substituents;
   reacting the silyl substituents on the Te to form Te-H bonds with (i) water and/or (ii) an alcohol having the general formula of ROH, where R is a C₁-C₁₀ alkyl group, a C₂-C₁₀ alkenyl group, a C₃-C₁₀ cyclic alkyl group, a C₃-C₁₀ cyclic alkenyl group, or a C₄-C₁₀ aromatic group;
   introducing into the deposition chamber a silylbismuth precursor selected from the group consisting of (D), to form a Bi layer on top of the Te layer, wherein the Bi comprises silyl substituents; and
   reacting the substituents on the Bi to form Bi-H bonds with (i) water and/or (ii) an alcohol having the general formula of ROH as defined above.
#2. The process of #1 wherein the alcohol is methanol.

## Claims

1. An ALD or CVD process for making an antimony- or bismuth-containing film on a surface of a substrate, the process comprising the steps of:
introducing into a deposition chamber a silylantimony or silylbismuth precursor selected from 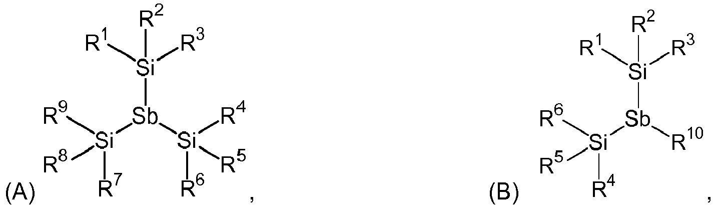 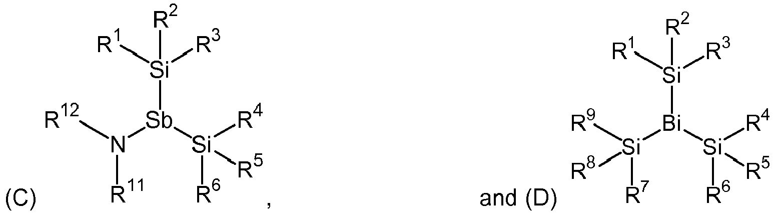 where R¹⁻¹⁰ are independently selected from hydrogen, a C₁-C₁₀ alkyl group, a C₂-C₁₀ alkenyl group, a C₃-C₁₀ cyclic alkyl group, a C₃-C₁₀ cyclic alkenyl group, or a C₄-C₁₀ aromatic group; R¹¹ and R¹² are independently selected from a C₁-C₁₀ alkyl group, a C₂-C₁₀ alkenyl group, a C₃-C₁₀ cyclic alkyl group, a C₃-C₁₀ cyclic alkenyl group, or a C₄-C₁₀ aromatic group to form a silylantimony or silylbismuth monolayer; and
introducing into the deposition chamber a second precursor selected from:
(a) M(OR¹³)₃, wherein M is selected from Ga, In, Sb and Bi; and R¹³ is a C₁-C₁₀ alkyl group, a C₂-C₁₀ alkenyl group, a C₃-C₁₀ cyclic alkyl group, a C₃-C₁₀ cyclic alkenyl group, or a C₄-C₁₀ aromatic group,
(b) M(OR¹³)₃₋ₓLₓ, wherein M is selected from Sb and Bi; L is selected from Cl, Br, I, or mixtures thereof; x is 0, 1 or 2 with a proviso that x cannot be 0 when M is Sb; and R¹³ is as defined above,
(c) M(OR¹⁴)₄₋ₓLₓ, wherein M is selected from Ge, Sn and Pb; L is as defined above; x is 0, 1, 2 or 3; R¹⁴ is a C₁-C₁₀ alkyl group, a C₂-C₁₀ alkenyl group, a C₃-C₁₀ cyclic alkyl group, a C₃-C₁₀ cyclic alkenyl group, or a C₄-C₁₀ aromatic group,
(d) M(NR¹⁴R¹⁵)₃₋ₓLₓ wherein M is selected from Sb, Bi, Ga and In; L is as defined above; x is 1, 2 or 3; R¹⁴ is a C₁-C₁₀ alkyl group, a C₂-C₁₀ alkenyl group, a C₃-C₁₀ cyclic alkyl group, a C₃-C₁₀ cyclic alkenyl group, or a C₄-C₁₀ aromatic group; and R¹⁵ is selected from hydrogen, a C₁-C₁₀ alkyl group or C₂-C₁₀ alkenyl group, a C₃-C₁₀ cyclic group, a C₃-C₁₀ cyclic alkenyl group, or a C₄-C₁₀ aromatic group,
(e) M(NR¹⁴R¹⁵)₄₋ₓLₓ wherein M is selected from Ge, Sn and Pb; L is as defined above; x is 1, 2 or 3; R¹⁴ is a C₁-C₁₀ alkyl group, a C₂-C₁₀ alkenyl group, a C₃-C₁₀ cyclic alkyl group, a C₃-C₁₀ cyclic alkenyl group, or a C₄-C₁₀ aromatic group; and R¹⁵ is selected from hydrogen, a C₁-C₁₀ alkyl group, a C₂-C₁₀ alkenyl group, a C₃-C₁₀ cyclic group, a C₃-C₁₀ cyclic alkenyl group, or a C₄-C₁₀ aromatic group, and
(f) [In(OCH₂CH₂NMe₂)₃]₂, In[N(^{t}Bu)(SiMe₃)]₃, In(TMP)₃ (TMP=2,2,6,6-tetramethylpiperidino), and In(N(cyclohexyl)₂)₃.

2. The process of claim 1, wherein the second precursor is selected from:
(a) M(OR¹³)₃, wherein M is selected from Ga and In; and R¹³ is a C₁-C₁₀ alkyl group, a C₂-C₁₀ alkenyl group, a C₃-C₁₀ cyclic alkyl group, a C₃-C₁₀ cyclic alkenyl group, or a C₄-C₁₀ aromatic group,
(c) M(OR¹⁴)₄₋ₓLₓ, wherein M is selected from Ge, Sn and Pb; L is as defined above; x is 0, 1, 2 or 3; R¹⁴ is a C₁-C₁₀ alkyl group, a C₂-C₁₀ alkenyl group, a C₃-C₁₀ cyclic alkyl group, a C₃-C₁₀ cyclic alkenyl group, or a C₄-C₁₀ aromatic group,
(d) M(NR¹⁴R¹⁵)₃₋ₓLₓ wherein M is selected from Ga and In; L is as defined above; x is 1, 2 or 3; R¹⁴ is a C₁-C₁₀ alkyl group, a C₂-C₁₀ alkenyl group, a C₃-C₁₀ cyclic alkyl group, a C₃-C₁₀ cyclic alkenyl group, or a C₄-C₁₀ aromatic group; and R¹⁵ is selected from hydrogen, a C₁-C₁₀ alkyl group or C₂-C₁₀ alkenyl group, a C₃-C₁₀ cyclic group, a C₃-C₁₀ cyclic alkenyl group, or a C₄-C₁₀ aromatic group,
(e) M(NR¹⁴R¹⁵)₄₋ₓLₓ wherein M is selected from Ge, Sn and Pb; L is as defined above; x is 1, 2 or 3; R¹⁴ is a C₁-C₁₀ alkyl group, a C₂-C₁₀ alkenyl group, a C₃-C₁₀ cyclic alkyl group, a C₃-C₁₀ cyclic alkenyl group, or a C₄-C₁₀ aromatic group; and R¹⁵ is selected from hydrogen, a C₁-C₁₀ alkyl group, a C₂-C₁₀ alkenyl group, a C₃-C₁₀ cyclic group, a C₃-C₁₀ cyclic alkenyl group, or a C₄-C₁₀ aromatic group, and
(f) [In(OCH₂CH₂NMe₂)₃]₂, In[N(^{t}Bu)(SiMe₃)]₃, In(TMP)₃ (TMP=2,2,6,6-tetramethylpiperidino), and In(N(cyclohexyl)₂)₃.

3. The process of claim 1 wherein the second precursor is selected from SbCl(OMe)₂, SbCl₂(OMe), SbBr(OMe)₂, SbBr₂(OMe), SbI(OMe)₂, SbCl(OEt)₂, SbCl₂(OEt), SbCl(OPrⁱ)₂, SbCl₂(OPrⁱ), BiCl(OMe)₂, BiCl₂(OMe), BiCl(OEt)₂, BiCl₂(OEt), BiCl(OPrⁱ)₂, BiCl₂(OPrⁱ), Cl₂SbNMeEt (II), Cl₂SbNEt₂ (III), ClSb[NMe₂]₂ (IV), ClSb[NMeEt]₂ (V), ClSb[NEt₂]₂ (VI), Ga(NMe₂)₂Cl, Ga(NMe₂)Cl₂, [In(µ-O^{t}Bu)(O^{t}Bu)₂]₂, and [In(OCMe₂Et)₂(m-OcMe₂Et)]₂.

4. An ALD or CVD process for making an antimony-containing film on a surface of a substrate, the process comprising the steps of:
introducing into a deposition chamber a germanium alkoxide as a precursor wherein the germanium alkoxide is represented by the formula Ge(OR¹⁴)₄ wherein R¹⁴ is selected from a C₁-C₁₀ alkyl group, a C₂-C₁₀ alkenyl group, a C₃-C₁₀ cyclic alkyl group, a C₃-C₁₀ cyclic alkenyl group, or a C₄-C₁₀ aromatic group, to form a molecular layer of germanium alkoxide on the surface of the substrate; and
introducing into the deposition chamber a silylantimony precursor selected from the group consisting of (A), (B) and (C) as defined in Claim 1, to form an Sb layer on top of the molecular layer of germanium alkoxide, wherein the Sb comprises silyl substituents.

5. A silylbismuth precursor selected from: 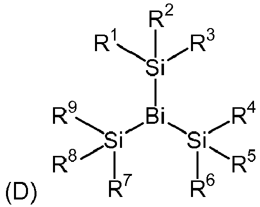 where R¹⁻⁹ are independently selected from hydrogen, a C₁-C₁₀ alkyl group, a C₂-C₁₀ alkenyl group, a C₃-C₁₀ cyclic alkyl group, a C₃-C₁₀ cyclic alkenyl group, or a C₄-C₁₀ aromatic group.

6. The silylbismuth precursor of Claim 5 selected from tris(trimethylsilyl)bismuth, tris(triethylsilyl)bismuth, and tris(tert-butyldimethylsilyl)bismuth.

7. A silylantimony precursor selected from tris(triethylsilyl)antimony and tris(tertbutyldimethylsilyl)antimony.

8. An ALD or CVD process for making a germanium-antimony-tellurium (GST) or germanium-bismuth-tellurium (GBT) film on a surface of a substrate, the process comprising the steps of:
introducing into a deposition chamber a germanium precursor selected from Ge(OR¹⁴)₄₋ₓLₓ, wherein L is selected from Cl, Br, I, or mixtures thereof; x is 0, 1, 2 or 3; R¹⁴ is a C₁-C₁₀ alkyl group, a C₂-C₁₀ alkenyl group, a C₃-C₁₀ cyclic alkyl group, a C₃-C₁₀ cyclic alkenyl group, or a C₄-C₁₀ aromatic group;
introducing into the deposition chamber a silyltellurium precursor selected from: 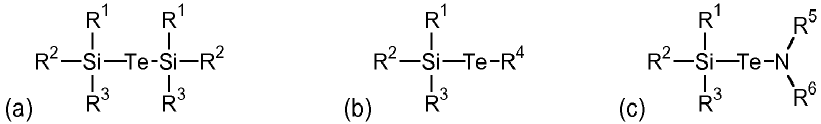 where R¹, R², R³, R⁴, R⁵, and R⁶ are independently selected from hydrogen, a C₁-C₁₀ alkyl group, a C₂-C₁₀ alkenyl group, a C₃-C₁₀ cyclic alkyl group, a C₃-C₁₀ cyclic alkenyl group, or a C₄-C₁₀ aromatic group;
introducing into the deposition chamber a germanium precursor as defined above;
introducing into the deposition chamber a silylantimony or silylbismuth precursor selected from: 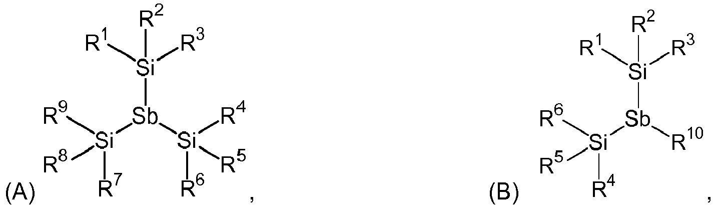 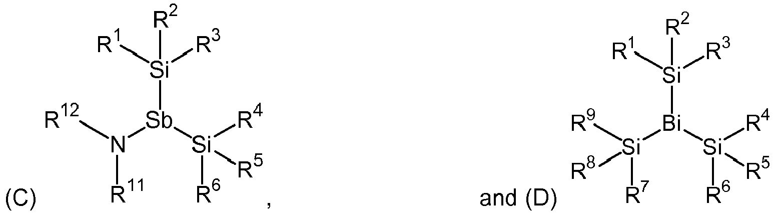 where R¹⁻¹⁰ are independently selected from hydrogen, a C₁-C₁₀ alkyl group, a C₂-C₁₀ alkenyl group, a C₃-C₁₀ cyclic alkyl group, a C₃-C₁₀ cyclic alkenyl group, or a C₄-C₁₀ aromatic group; R¹¹ and R¹² are independently selected from a C₁-C₁₀ alkyl group, a C₂-C₁₀ alkenyl group, a C₃-C₁₀ cyclic alkyl group, a C₃-C₁₀ cyclic alkenyl group, or a C₄-C₁₀ aromatic group, to form a silylantimony or silylbismuth monolayer; and
repeating the steps above until a desired thickness is reached.

9. The process of claim 8 wherein the germanium precursor is selected from Ge(OMe)₄, Ge(OEt)₄, Ge(OPrⁿ)₄, Ge(OPrⁱ)4, GeCl(OMe)₃, GeCl₂(OMe)₂, GeCl₃(OMe), GeCl(OEt)₃, GeCl₂(OEt)₂, GeCl₃(OEt), GeCl(OPrⁿ)₃, GeCl(OPrⁿ)₃, GeCl₂(OPrⁿ))₂, GeCl₂( OPrⁱ)₂, GeCl₃(OPrⁱ), GeCl(OBu^{t})₃, GeCl₂(OBu^{t}))₂, and GeCl₃(OBu^{t}).

10. The process of any one of claims 1-4 and 8-9 wherein the silylantimony precursor is selected from tris(trimethylsilyl)antimony, tris(triethylsilyl)antimony, tris(tertbutyldimethylsilyl)antimony, and tris(dimethylsilyl)antimony and/or the silylbismuth precursor is selected from tris(trimethylsilyl)bismuth, tris(triethylsilyl)bismuth, and tris(tert-butyldimethylsilyl)bismuth.

11. The process of claim 8 or 9 wherein the disilyltellurium precursor is selected from the group consisting of bis(trimethylsilyl)tellurium, bis(triethylsilyl)tellurium, and bis(tertbutyldimethylsilyl)tellurium.

12. The process of any one of claims 1-4 and 8-11 wherein the steps are repeated in sequence.

13. The process of any one of claims 1-4 and 8-12 wherein the temperature of the deposition chamber is from room temperature to 400°C.
